(19) Europäisches Patentamt
European Patent Office
Office européen des brevets

(11) **EP 2 263 533 A1**

(12) **EUROPEAN PATENT APPLICATION**

(43) Date of publication:
**22.12.2010 Bulletin 2010/51**

(51) Int Cl.:
***A61B 5/11*** *(2006.01)* ***H04M 1/725*** *(2006.01)*

(21) Application number: **09163074.9**

(22) Date of filing: **18.06.2009**

<table>
<tr><td>(84) Designated Contracting States:<br>**AT BE BG CH CY CZ DE DK EE ES FI FR GB GR HR HU IE IS IT LI LT LU LV MC MK MT NL NO PL PT RO SE SI SK TR**</td><td>• **Technische Universität Berlin**<br>**10623 Berlin (DE)**</td></tr>
<tr><td>(71) Applicants:<br>• **Deutsche Telekom AG**<br>**53113 Bonn (DE)**</td><td>(72) Inventor: **Ketabdar, Hamed**<br>**10713 Berlin (DE)**<br><br>(74) Representative: **Vossius & Partner**<br>**Siebertstrasse 4**<br>**81675 München (DE)**</td></tr>
</table>

(54) **Enhancing security and emergency functionalities in mobile phones based on detection of physical shocks**

(57) The invention provides a method and mobile phone of identifying at least one of a security or emergency critical situation of a user of a mobile phone, the mobile phone comprising at least one acceleration sensor, the method comprising the steps of: obtaining at least one acceleration signal from said at least one acceleration sensor representing a current situation of the mobile phone; pre-processing said obtained at least one acceleration signal to remove redundant information present in said at least one signal; performing a feature extraction on said pre-processed at least one acceleration signal; performing a shock detection based on the acceleration pattern represented by the extracted acceleration features; and performing a subsequent mobile phone activity detection if a shock is detected in step (d); wherein a security or emergency critical situation is identified if the shock is followed by a period of no or low activity.

EP 2 263 533 A1

**Description**

Field of Invention

The invention provides a method which operates on a mobile or portable phone/telephone and uses accelerometers integrated in the mobile phone as well as optional audio information to detect a physical shock experienced by the mobile phone or its user. Detection of shock is then used to identify security and emergency critical situations.

Background of the invention

Unexpected events such as physical shocks can be signs of critical security or emergency scenarios, and thus very important to detect. For detecting such events, usually there is a need for a device with a sense of physical variables (such as acceleration). This device should be convenient to carry by the user, for instance a mobile phone. Over the past few years, many mobile phones are equipped with new sensors such as acceleration sensors. A physical shock can show itself by a rapid and huge change in the pattern of acceleration sensed by mobile phone accelerometers. In addition, audio information provided by microphone can also help for detecting an impact.

Detecting a shock by a mobile phone can have several applications. It can be used to detect unexpected scenarios such as 'a mobile phone has fallen'. A mobile phone which is fallen and left unattended for a while can be under the risk of being lost. Considering that this mobile phone can contain private or confidential data, detecting such event can be essential for security purposes. Detecting a physical shock can be also useful in emergency scenarios such as detecting an accident involving the user of the mobile phone. Some accidents such as a car accident or a person falling can be identified by a sudden shock, and subsequently sudden change in pattern of acceleration sensed by the mobile phone. The mobile phone can then automatically inform a designated emergency institution for help.

JP-A-2003-219061 describes a mobile phone with an emergency message function. It describes to use accelerometer and microphone in a mobile phone to detect shock. It stores a prescribed level corresponding to the magnitude of the detected impact force or shock sound, and compares a prescribed stored level with the magnitude of the detected impact force or shock sound. The emergency messaging function is configured to make an emergency messaging depending on the result of the comparison.

EP-A-1 109 378 discloses monitoring the shock to which a mobile phone is subjected for warranty purposes. According to EP-A-1 109 378, the dynamic speaker provided as standard equipment in many items of mobile electronic apparatus such as mobile phones is used as a transducer to sense the shock experienced by the phone. The shock signal recorded by microphone can be accessed during service to determine if the phone was subjected to excessive force which may negate the warranty.

US-A-6 453 266 discloses a method and structure for preventing damage to an electronic device. The structure includes a sensor outputting signals indicating environmental conditions experienced by the electronic device, a non-volatile memory storing ones of the signals that exceed a limit, and an output device outputting signals stored in the non-volatile memory, thereby providing a history of the environmental conditions experienced by the electronic device that exceed the limit.

US-A-4 823 290 describes a method and apparatus for a system for automatically monitoring the operating environment and other physical conditions around and in which a host computer system operates. Sensors located around a computer system, including inside equipment cabinets and under the floor where cabling and air are ducted, are used to collect data that is frequently and periodically collected and stored along with an indication of the time and data of collection.

Summary of the Invention

Detecting shock as an usual event can have several applications considering emergency and security risky situations. In general terms, the invention provides a mechanism which enables a mobile phone to detect a physical shock experienced by the mobile phone or its user. The invention also provides to use this shock detection mechanism for different security and emergency related applications.

A shock is usually associated with rapid and huge change in pattern of physical variables such as acceleration. Today, more and more mobile phones are equipped with embedded accelerometer sensors. In case of shock experienced either by the mobile phone or its user, the acceleration pattern sensed by mobile phone's accelerometers is affected. The invention provides an intelligent algorithm based on sophisticated signal processing and machine learning techniques to detect the acceleration pattern associated with a shock and to distinguish it form the rest of possible usual activities. In case of a shock, audio data captured by microphone shows also a rapid change towards an impact sound. According to a preferred embodiment of the invention, audio data are additionally used for detection of shock and its associated security and emergency issues.

As mentioned above, the shock can be an indication of several critical security and emergency related situations. One application encompassed by the invention related to security of data on mobile phones is preventing a mobile phone to be lost or stolen. Today, as the mobile phones are improved in terms of user interface, access and storage capacity, there are more and more private or company related data which are stored or accessed through them. If such a mobile phone is lost or stolen, there is a high risk that this private or confidential data can be exposed. In many scenarios, a user may loose a mobile phone, when the phone drops accidentally out of his pocket/bag and left unattended for a while. When the phone drops on a surface, it experiences a serious shock, and may be left unattended for a while. Such a shock followed by no activity (corresponding to the phone being unattended), changes the acceleration pattern (sensed by mobile phone's accelerometers) as well audio pattern accordingly. The invention provides for detecting such scenario. When such a case is detected, it is preferred according to the invention that the mobile phone operating system locks the mobile phone and asks for the entry of a password to restore access to the mobile phone. Detecting the possibility that the cell phone is unattended or lost enables the phone to be locked and/or restrict access to certain data and functionalities unless a certain unlocking code is entered. In this way, if the mobile phone is lost or stolen, the risk of access to private and confidential data is substantially reduced. In addition or alternatively, when such a scenario is detected, the mobile phone preferably issues an alarm and/or automatically reports its location and situation to a designated centre, in order to facilitate finding the mobile phone.

The invention also encompasses the use of a mobile phone with a shock detection ability to detect unexpected situations experienced by the user. For instance, many accidents such as falling, car accidents, etc., involves a rapid shock (rapid change in acceleration pattern) followed by no physical activity. These scenarios are detectable by the method of the invention. Preferably, the mobile phone then automatically informs a designated organization or person such as emergency, police, etc. by sending a message and/or making a call reporting the current position. Such a system can be very crucial, considering the case that the accident has happened in a deserted place, where no body can observe it. It can be also very useful for elderly or people with high risk of fall to be constantly and precisely monitored, and saved quickly if necessary.

According to a first aspect, the invention provides a method of identifying at least one of a security or emergency critical situation of a user of a mobile phone, the mobile phone comprising at least one acceleration sensor, the method comprising the steps of:

(a) obtaining at least one acceleration signal from the at least one acceleration sensor representing a current situation of the mobile phone;
(b) pre-processing the obtained at least one acceleration signal to remove redundant information present in the at least one signal;
(c) performing a feature extraction on the pre-processed at least one acceleration signal;
(d) performing a shock detection based on the acceleration pattern represented by the extracted acceleration features; and

(e) performing a subsequent mobile phone activity detection if a shock is detected in step (d); wherein a security or emergency critical situation is identified based on the performed activity detection.

The step (d) of performing a shock detection preferably comprises comparing the acceleration pattern with at least one statistical reference model for different conditions. The at least one statistical reference model represents normal or regular conditions. The detected current situation is classified as normal situation or shock situation based on the comparison of the acceleration pattern with at least one statistical reference model. The statistical model is preferably an artificial neural network or a Gaussian mixture model.

The features extracted from the pre-processed at least one acceleration signal are preferably selected from the group comprising: acceleration magnitude over different axes, the rate of change in acceleration over different axis, the absolute magnitude of the acceleration, and pairwise difference between acceleration magnitudes over different axis.

According to the invention, step (e) determines whether there is a pre-defined period of the mobile phone of no or low physical activity or no or low rate of change in acceleration detected by at least one of said acceleration sensors. In order to detect no activity period, maximum of acceleration values in an interval is compared to a prescribed threshold.

It is preferred according to the invention that an operating state of the mobile phone is changed if security or emergency critical situation is identified in step (e). For example, the operating system of the mobile phone is initiated dependent on the detected shock and period of no activity to switch to an operating mode of limited or restricted access to the mobile phone. This represents a security application. Alternatively, the transmission of an emergency warning is automatically initiated dependent on the detected shock and period of no activity. This represents an emergency related application.

According to a preferred embodiment, step (a) further comprises obtaining an audio signal from the microphone of said mobile phone, said audio signal representing environmental sound of the mobile phone, step (b) further comprises pre-processing said obtained audio signal to remove redundant information present in said signal, step (c) further comprises performing a feature extraction on said pre-processed audio signal, and wherein step (d) performs said shock detection also based on the audio pattern represented by the extracted audio features. The features extracted from said pre-processed audio signal are selected from the group comprising: magnitude, loudness, energy over different frequency bands, and periodicity or pitch.

According to a second aspect, the invention provides a mobile phone comprising at least one acceleration sensor providing at least one acceleration signal to a processor. The processor is configured for pre-processing the at least one acceleration signal to remove redundant information present in said signals, for performing a feature extraction on the pre-processed at least one acceleration signal; for performing a shock detection based on the acceleration pattern represented by the extracted acceleration features, and for performing a subsequent mobile phone activity detection if a shock is detected; wherein a security or emergency critical situation is identified based on the performed activity detection, e.g. if the shock is followed by no activity. According to a third aspect, the invention provides a computer program product comprising instructions for performing the method of the first aspect of the invention.

Although the invention is described in relation to a mobile phone, other devices such as palm, handheld, or PDAs are also encompassed by the invention.

Other aspects, features, and advantages will be apparent from the summary above, as well as from the description that follows, including the figures and the claims.

The invention will now be described with reference to the accompanying drawings which show in

Fig. 1    a general overview of the system according to the invention;

Fig. 2    an example of acceleration signals along x, y, and z axis, when a mobile phone drops from user hand on the floor; and

Fig. 3    for the example of Fig. 2, the acceleration signals after being post-processed by a high pass filter.

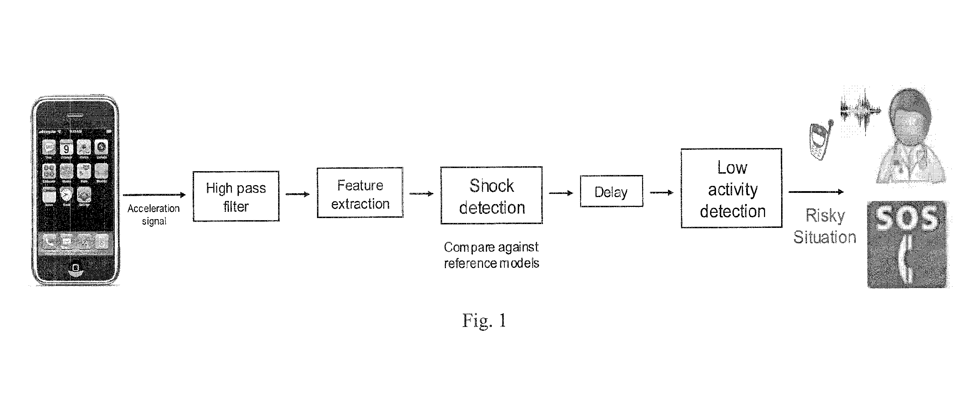

Fig. 1 shows a general overview of the system according to the invention. Fig. 1 shows a mobile phone having acceleration sensors sensing acceleration along x, y, and z axis. It is preferred that the acceleration pattern along each axis is captured constantly within certain snapshots (e.g. 20 ms). As shown in Fig. 1, the acceleration data is first passed through a pre-processer, in the shown preferred embodiment a high pass filter to remove DC and low frequency component, and preserve acceleration pattern caused by a shock which has higher frequency components. This is especially important since the algorithm should not react to any source of huge acceleration. For instance, if the user is in a metro or normally driving a car, accelerometers may still sense huge acceleration (due to the movement of vehicle). However, such acceleration pattern changes more slowly than the acceleration pattern associated with a sudden physical shock.

After pre-processing the acceleration signal using a high pass filter, a feature extraction is performed. Feature extraction removes redundant information in the acceleration pattern and preserves information which can represent the shock pattern in a more discriminative way. According to the invention, feature extraction methods are used which are not computationally expensive considering limited computational resources available in a mobile phone. All the feature extraction methods are applied to a time window of the acceleration signal. This window can be 200-400ms seconds long. The value of extracted features is averaged over samples in each window. Adjacent windows can have overlaps up to 50%.

Preferably, magnitude of acceleration along different axis, rate of change in acceleration along different axis, or absolute magnitude and rate of change in acceleration are used as features in the feature extraction:

1. Acceleration magnitude over different axis: This is absolute value of acceleration over different x, y, and z axis, i.e. $|a_x|$, $|a_y|$, $|a_z|$, where $a$ indicates acceleration

2. Rate of change of acceleration over different axis: This is derivate of acceleration signal (over different axis) with respect to time, i.e. $da_x/dt$, $da_y/dt$, $da_z/dt$.

3. Absolute magnitude of acceleration: This is defined as:

$$a = \sqrt{a_x^2 + a_y^2 + a_z^2}$$

Further preferred is

4. Pairwise difference between acceleration magnitudes along different axis, i.e. $|a_x - a_y|$,

$$\left|a_x - a_z\right|, \left|a_y - a_z\right|$$

However, it is also preferred that combinations or even all of these types are applied together.

As mentioned before, audio data captured by microphone can be also used in shock detection process, as a shock is associated with high audio activity pattern. In case audio data are additionally taken into consideration, pre-processing is also performed on the audio data, followed by feature extraction. The following audio based features are preferred according to the invention:

1. Magnitude: This is defined as average energy of the audio signal samples in a limited time window (200-400 ms) around the current time sample.

2. Energy over different frequency bands: This is defined as average energy of audio signal samples in a limited window (200-400 ms around current time sample) within a certain frequency range.

3. Periodicity or pitch: This is defined as fundamental frequency component of audio signal in a limited time window (200-400 ms around current time sample).

Further details on extraction of audio based features can be found in Fundamcentals of Speech Recognition; Lawrence Rabiner & Biing-Hwang Juang Englewood Cliffs NJ: PTR Prentice Hall (Signal Processing Series), c1993, ISBN 0-13-015157-2.

According to the invention, the extracted features are used for training reference statistical models for two classes: 1) normal conditions and 2) shock. Having the reference models, the invention matches new samples of acceleration data against reference models and classifies the ongoing scenario as shock or normal condition. An alternative would be training only one model for normal condition and measuring a score for matching between new data and the normal condition model. A low score corresponding to a low match can indicate a shock situation.

As mentioned above, in a situation which can be risky concerning the mobile phone being lost, or an accident happened for the phone's user, a shock is usually followed by a period of 'low activity'. This means that the mobile phone or its user is left unattended or unobserved. As shown in Fig. 1, when a shock is detected by the mobile phone, it is verified if the shock is followed by a period of low activity or low rare of change in acceleration. If the shock is followed by a certain period of low activity, it indicates that the shock indeed corresponds to a risky situation. This allows avoiding false alarms, for instance when a mobile phone accidently falls out of the user hands, but picked up immediately. In this case, since the shock is not followed by having the phone unattended (because it is picked up), there will be no warning issued.

As shown in Fig. 1, if both conditions, i.e. initially a shock and then followed by a period of low activity is detected, a warning or action is issued. In case of a security application, the mobile phone operation system may block or restrict the access to some data or services existing in the mobile phone unless a password in entered, as mentioned above. It can additionally issue and alarm, send its location to a designed centre, or automatically send short text or voice message to a designated number. In case of emergency related application, the mobile detects that an accident (e.g. car crash, fall) has happened for the user, issues an alarm and automatically informs a designated centre such as medical emergency, police, a friend, etc. about the location and time of the accident by a text or voice message.

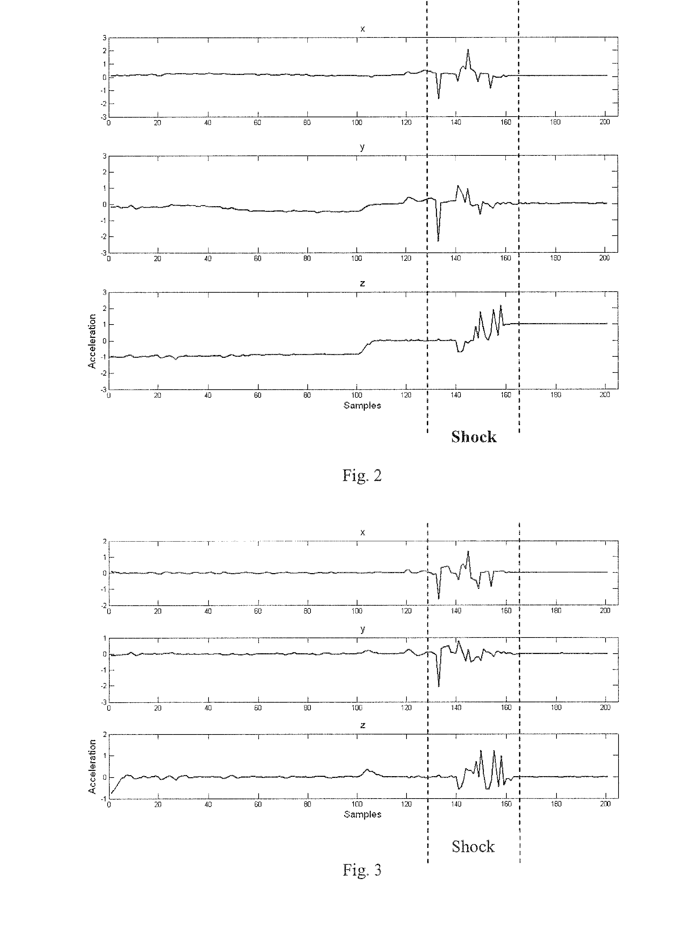

Fig. 2 shows an example of acceleration signals along x, y, and z axis, when a mobile phone drops from user hand on the floor. The pattern corresponding to the shock is marked in the figure. The y-axis in the plots shows value of acceleration and the x-axis shows samples (time).

Fig. 3 shows for the example of Fig. 2 the acceleration signals along x, y, and z axis after being processed by a high pass filter. Again, the pattern corresponding to the shock is marked in the figure.

Example

The following describes an experiment for detecting physical shock by a mobile phone according to the present invention. An Apnle™ iPhone™ 3G was used as the mobile phone. Acceleration signal is provided along x, y, and z axis by accelerometers integrated in iPhone. The acceleration signal is processed according to the invention.

For the experiments, a database of normal and shock situations was collected. In this database, there are 65 samples of normal situations, and 24 samples of physical shock. In order to obtain physical shock, the iPhone was allowed to fall on a carpet or wooden floor from a distance of approximately 35 cm. In order to obtain normal condition samples, 5 users carried the phone normally in their pocket, hand or bag for a period of 10 seconds. These users do different normal activities such as walking, judging, taking stairs, and taking elevator. A variety of scenarios was aimed at, especially those which can have similarity to a shock (due to high physical activity) such as taking stairs or elevator. In this way, it was ensured that the invention is able to distinguish between such cases and a real shock.

The obtained data was used to train reference statistical models for the two cases of normal and shock situations. As statistical model, Gaussian Mixture Models (GMMs) (McLachlan, G.J. and Basford, K.E. "Mixture Models: Inference and Applications to Clustering", Marcel Dekker (1988)) were used with three Gaussians for each situation (class).

During the test of the system, the acceleration data is matched against the reference models for shock and normal situations. If the shock model gives a better match (higher likelihood score) compared to the normal situation model, the case is classified as a shock and a warning is issued. Table 1 summarizes the initial results on detection of shock. True alarm is when a shock is correctly detected. False alarm is when a shock is detected but it is normal situation.

**Table 1. Initial results on detection of shock**

| Accuracy | True | False |
|----------|------|-------|
| 91.1%    | 21   | 5     |

The present invention is different to conventional approaches in many aspects.

For example, with respect to aforementioned JP-A-2003-219061, the invention is different in at least two aspects. First, the method for detecting the shock according to the invention is not only based on comparing the magnitude of acceleration or audio signal with a prescribed level. In the invention, the pattern of acceleration signal is analysed and matched against statistical models for shock pattern. Therefore, the detection of shock is not only based on magnitude of acceleration or audio signal, but based on certain characteristics of impact (shock) in frequency domain. This allows avoiding many false alarms. A high magnitude of acceleration or voice activity can happen due to different situations other than a shock such as being in a car, in a party or celebration, thus only relying on magnitude of acceleration or audio signal is not enough. In addition, method of the invention also checks for pattern of an acceleration signal after a potential shock. Any kind of shock is not a sign of critical security or emergency situation. If the shock is not followed by a period of 'low activity', corresponding to have the mobile phone or its user unattended or unobserved, no alarm will be issued. This also helps to avoid false alarms for instance if the phone falls out of user's hand accidentally but picked up immediately. The second aspect of difference is the application of shock detection in increasing the security of data access in mobile phones. This aspect allows detecting if the phone is under the risk of being lost or stolen and will restrict the access to data and services through the phone if such cases happen.

**Claims**

1.  Method of identifying at least one of a security or emergency critical situation of a user of a mobile phone, the mobile phone comprising at least one acceleration sensor, the method comprising the steps of:

    (a) obtaining at least one acceleration signal from said at least one acceleration sensor representing a current situation of the mobile phone;
    **characterized by**
    (b) pre-processing said obtained at least one acceleration signal to remove redundant information present in said at least one signal;
    (c) performing a feature extraction on said pre-processed at least one acceleration signal;
    (d) performing a shock detection based on the acceleration pattern represented by the extracted acceleration features; and
    (e) performing a subsequent mobile phone activity detection if a shock is detected in step (d); wherein a security or emergency critical situation is identified based on the performed activity detection.

2.  The method of claim 1, wherein step (d) of performing a shock detection comprises comparing said acceleration pattern with at least one statistical reference model for different conditions.

3.  The method of claim 2, wherein said at least one statistical reference model represents normal or regular conditions.

4.  The method of claim 2 or 3, further comprising classifying the detected current situation as normal situation or shock situation based on the comparison of the acceleration pattern with at least one statistical reference model.

5.  The method of claim 2 or 3, further comprising measuring a score for matching between the detected current situation and normal condition model.

6.  The method of any of claims 2 to 5, wherein said statistical model is an artificial neural network or a Gaussian mixture model.

7.  The method of any of the preceding claims, wherein features extracted from said pre-processed at least one acceleration signal are selected from the group comprising: acceleration magnitude over different axes, the rate of change in acceleration over different axis, the absolute magnitude of the acceleration, and pairwise difference between acceleration magnitudes over different axis.

8.  The method of any of the preceding claims, wherein step (e) determines whether there is a pre-defined period of the mobile phone of no or low physical activity or no or low rate of change in acceleration detected by at least one of said acceleration sensors.

9.  The method of any of the preceding claims, further comprising the step of changing an operating state of the mobile phone if security or emergency critical situation is identified in step (e).

10. The method of any of the preceding claims, wherein the transmission of an emergency warning is automatically initiated dependent on the detected shock followed by no activity.

11. The method of any of claims 1 to 9, wherein the operating system of the mobile phone is initiated to switch to an operating mode of limited or restricted access to the mobile phone.

12. The method of any of the preceding claims, wherein step (a) further comprises obtaining an audio signal from the microphone of said mobile phone, said audio signal representing environmental sound of the mobile phone, step (b) further comprises pre-processing said obtained audio signal to remove redundant information present in said signal, step (c) further comprises performing a feature extraction on said pre-processed audio signal, and wherein step (d) performs said shock detection also based on the audio pattern represented by the extracted audio features.

13. The method of claim 12, wherein features extracted from said pre-processed audio signal are selected from the group comprising: magnitude, loudness, energy over different frequency bands, and periodicity or pitch.

14. Mobile phone comprising
at least one acceleration sensor providing at least one acceleration signal to a processor; **characterized by**
said processor being configured for pre-processing said at least one acceleration signal to remove redundant information present in said signals, being further configured for performing a feature extraction on said pre-processed at least one acceleration signal; being further configured for performing a shock detection based on the acceleration pattern represented by the extracted acceleration features, and being further configured for performing a subsequent mobile phone activity detection if a shock is detected; wherein a security or emergency critical situation is identified based on the performed activity detection.

15. Computer program product, comprising instructions for performing the method of any of claims 1 to 13.

8

Acceleration signal →

High pass filter →

Feature extraction →

Shock detection →

Delay →

Low activity detection →

Risky Situation

Compare against reference models

SOS

Fig. 1

Fig. 2

Fig. 3

Europäisches
Patentamt
European
Patent Office
Office européen
des brevets

**EUROPEAN SEARCH REPORT**

Application Number

EP 09 16 3074

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>A | US 2006/161079 A1 (CHOI JI-HYUN [KR] ET AL) 20 July 2006 (2006-07-20)<br><br>* paragraph [0057] - paragraph [0061] *<br>* paragraph [0069] - paragraph [0071] *<br>* paragraph [0052] *<br>* paragraph [0029] *<br>* claim 17 * | 1,3,5,<br>7-8,10,<br>14-15<br>2,4,6,9,<br>11 | INV.<br>A61B5/11<br>H04M1/725 |
| X<br><br>A | CH 697 402 B1 (LIFEJOB AG [CH]) 30 September 2008 (2008-09-30)<br>* paragraph [0007] - paragraph [0010] *<br>* paragraph [0020] - paragraph [0021] *<br>* paragraph [0022] *<br>* paragraph [0024] *<br>* paragraph [0027] *<br>* claim 1 * | 1-4,6-7,<br>14-15<br>5 | |
| X | US 2005/208925 A1 (PANASIK CARL M [US] ET AL) 22 September 2005 (2005-09-22)<br>* paragraph [0017] - paragraph [0034] *<br>* paragraph [0039] *<br>* paragraph [0047] - paragraph [0050] *<br>* paragraph [0043] * | 1,8,10,<br>14-15 | |
| X | US 2005/046580 A1 (MIRANDA-KNAPP CARLOS A [US] ET AL) 3 March 2005 (2005-03-03)<br>* paragraph [0017] *<br>* claim 1 * | 1,9,11 | |
| X,D | JP 2003 219061 A (NEC SAITAMA LTD) 31 July 2003 (2003-07-31)<br>* paragraph [0020] *<br>* paragraph [0027] *<br>* paragraph [0033] *<br>* paragraph [0043] * | 1,12-15 | |

TECHNICAL FIELDS
SEARCHED      (IPC)

A61B
H04M
G08B

-/--

The present search report has been drawn up for all claims

| Place of search | Date of completion of the search | Examiner |
|---|---|---|
| The Hague | 25 February 2010 | de la Cruz Valera, D |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

**EUROPEAN SEARCH REPORT**

Application Number

EP 09 16 3074

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/128062 A1 (LUNDSGAARD SOREN K [US] ET AL) 16 June 2005 (2005-06-16) | 1,14-15 | |
| A | * paragraph [0038] - paragraph [0040] * | 2-11 | |
| A | US 2002/118110 A1 (EKKEL FREDERIK [US]) 29 August 2002 (2002-08-29) * paragraph [0034] - paragraph [0035] * | 1-11, 14-15 | |
| A | WO 2006/101587 A2 (FREESCALE SEMICONDUCTOR INC [US]; CLIFFORD MICHELLE A [US]; BORRAS ROD) 28 September 2006 (2006-09-28) * paragraph [0054] * | 1,7-8,10 | |
| A | MIHAIL POPESCU ET AL: "An acoustic fall detector system that uses sound height information to reduce the false alarm rate" ENGINEERING IN MEDICINE AND BIOLOGY SOCIETY, 2008. EMBS 2008. 30TH ANNUAL INTERNATIONAL CONFERENCE OF THE IEEE, IEEE, PISCATAWAY, NJ, USA, 20 August 2008 (2008-08-20), pages 4628-4631, XP031347778 ISBN: 978-1-4244-1814-5 * page 4629, right-hand column - page 4630, left-hand column * | 12-13 | TECHNICAL FIELDS SEARCHED (IPC) |
| A | MIHAIL POPESCU ET AL: "A Fuzzy Logic System for Acoustic Fall Detection" PROCEEDINGS. 2008 AAAI SYMPOSIUM ON AI IN ELDERCARE,, 1 November 2008 (2008-11-01), pages 1-6, XP007908814 * page 3, left-hand column * | 13 | |

-/--

The present search report has been drawn up for all claims

| Place of search | Date of completion of the search | Examiner |
|---|---|---|
| The Hague | 25 February 2010 | de la Cruz Valera, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 09 16 3074

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHARALAMPOS DOUKAS ET AL: "Human Distress Sound Analysis and Characterization Using Advanced Classification Techniques" 2 October 2008 (2008-10-02), ARTIFICIAL INTELLIGENCE: THEORIES, MODELS AND APPLICATIONS; [LECTURE NOTES IN COMPUTER SCIENCE], SPRINGER BERLIN HEIDELBERG, BERLIN, HEIDELBERG, PAGE(S) 73 - 84 , XP019107502 ISBN: 9783540878803 * page 73, paragraph 2 - page 74, paragraph 1 * * page 75 * * page 77 * ----- | 12-13 | |

TECHNICAL FIELDS
SEARCHED      (IPC)

The present search report has been drawn up for all claims

| Place of search | Date of completion of the search | Examiner |
|---|---|---|
| The Hague | 25 February 2010 | de la Cruz Valera, D |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...........................................................................................
& : member of the same patent family, corresponding
document

EPO FORM 1503 03.82 (P04C01)

Europäisches
Patentamt
European
Patent Office
Office européen
des brevets

Application Number

EP 09 16 3074

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 09 16 3074

The Search Division considers that the present European patent application does not comply with the
requirements of unity of invention and relates to several inventions or groups of inventions, namely:

    1. claims: 1-11, 14-15

        Mobile phone alarm via acceleration detection.
           ---

    2. claims: 12-13

        Noise processing in mobile phone.
           ---

## ANNEX TO THE EUROPEAN SEARCH REPORT
## ON EUROPEAN PATENT APPLICATION NO.

EP 09 16 3074

This annex lists the patent family members relating to the patent documents cited in the above-mentioned European search report.
The members are as contained in the European Patent Office EDP file on
The European Patent Office is in no way liable for these particulars which are merely given for the purpose of information.

25-02-2010

| Patent document cited in search report | | Publication date | Patent family member(s) | | Publication date |
|---|---|---|---|---|---|
| US 2006161079 | A1 | 20-07-2006 | JP | 2006192276 A | 27-07-2006 |
| CH 697402 | B1 | 30-09-2008 | NONE | | |
| US 2005208925 | A1 | 22-09-2005 | NONE | | |
| US 2005046580 | A1 | 03-03-2005 | BR | PI0413893 A | 24-10-2006 |
| | | | EP | 1661099 A2 | 31-05-2006 |
| | | | JP | 2007504714 T | 01-03-2007 |
| | | | KR | 20060060714 A | 05-06-2006 |
| | | | WO | 2005025187 A2 | 17-03-2005 |
| JP 2003219061 | A | 31-07-2003 | NONE | | |
| US 2005128062 | A1 | 16-06-2005 | NONE | | |
| US 2002118110 | A1 | 29-08-2002 | WO | 02069293 A1 | 06-09-2002 |
| WO 2006101587 | A2 | 28-09-2006 | JP | 2008535055 T | 28-08-2008 |
| | | | US | 2006214806 A1 | 28-09-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003219061 A
- EP 1109378 A
- US 6453266 A
- US 4823290 A

**Non-patent literature cited in the description**

- **Lawrence Rabiner ; Biing-Hwang Juang.** Fundamcentals of Speech Recognition. PTR Prentice Hall, 1993
- **McLachlan, G.J. ; Basford, K.E.** Mixture Models: Inference and Applications to Clustering. Marcel Dekker, 1988